Europäisches Patentamt

European Patent Office

Office européen des brevets

(19)

(11) Veröffentlichungsnummer: **0 012 709**
**B1**

(12) **EUROPÄISCHE PATENTSCHRIFT**

(45) Veröffentlichungstag der Patentschrift: **29.02.84**

(51) Int. Cl.³: **H 03 G 3/20, A 61 B 5/04**

(21) Anmeldenummer: **79730010.0**

(22) Anmeldetag: **23.11.79**

(54) Schaltungsanordnung für ein elektrokardiographisches Signal.

(30) Priorität: **09.12.78 DE 2853642**

(43) Veröffentlichungstag der Anmeldung:
**25.06.80 Patentblatt 80/13**

(45) Bekanntmachung des Hinweises auf die Patenterteilung:
**29.02.84 Patentblatt 84/9**

(84) Benannte Vertragsstaaten:
**AT BE CH DE FR GB IT NL SE**

(56) Entgegenhaltungen:
DE - A - 2 032 102
DE - A - 2 113 815
FR - A - 2 105 692
FR - A - 2 129 053

INTERNATIONAL JOURNAL OF ELECTRONICS,
vol. 43, Heft 6, Dezember 1977, London GB
LARSEN und BESSLICH: "An improved receiver
for baseband data communication using a
digitally controlled AGC", Seiten 593-598

(73) Patentinhaber: BIOTRONIK Mess- und
Therapiegeräte GmbH & Co Ingenieurbüro Berlin
Sieversufer 8
D-1000 Berlin 47 (DE)

(72) Erfinder: Blaser, Reinhard, Dr.-Ing.
Binsenweg 16
D-8500 Nürnberg (DE)
Erfinder: Olach, Klaus
Kaiser-Wilhelm-Strasse 104
D-1000 Berlin 46 (DE)
Erfinder: Schaldach, Max, Prof. Dr.-Ing.
Turnstrasse 5
D-8520 Erlangen (DE)

(74) Vertreter: Christiansen, Henning, Dipl.-Ing.
Unter den Eichen 108a
D-1000 Berlin 45 (DE)

(56) Entgegenhaltungen:
IEEE TRANSACTIONS ON INDUSTRIAL
ELECTRONICS AND CONTROL
INSTRUMENTATION, vol. IECI-21, Heft 4,
November 1974, New York USA H. MADKOUR:
"A processing unit for wide-range measuring
channels" Seiten 230-234

Schaltungsanordnung für ein elektrokardiographisches signal

Beschreibung

Die Erfindung betrifft eine digital arbeitende Amplitudenregelschaltung für ein elektrokardiographisches Signal.

Bei einer Reihe von biomedizinischen Geräten werden elektrokardiographische Signale als Synchronisationssignale für elektrotherapeutische Maßnahmen bzw. zur Signalerkennung oder -darstellung benutzt. Die sich im Betrieb stark verändernden Verhältnissebedingt beispielsweise durch die Signalableitung über Elektroden—und die daraus resultierenden schwankenden Signalamplituden erfordern eine ständige Anpassung der Verstärkung im Übertragungskanal damit einerseits nicht bei einer Übertragung mit zu großer Verstärkung die im abgeleiteten Signal vorhandenen Störsignale mit Impulsen des QRS-Komplexes verwechselt werden und andererseits auch nicht durch zu kleine Verstärkung der QRS-Komplex in den Störsignalen untergeht bzw. die auftretenden Amplitudenspitzen nicht mehr zum Auslösen der Synchronisationsvorgänge ausreichen.

Die Erkennung der EKG-Signale ist nicht nur erschwert durch deren niedrige und wechselnde Frequenz, sondern wird zusätzlich beeinträchtigt durch auftretende Störsignale (Artefakte), deren Amplitude die der Nutzsignale oft um mehr als das Hundertfache übertrifft.

Aus der DE—OS 20 32 102 ist eine digital arbeitende Schaltungsanordnung zur automatischen Voreinstellung der Verstärkung eines Verstärkers für elektrokardiographische Signale bekannt, bei dem das Eingangssignal gleichgerichtet und einer Integrationsschaltung zugeführt wird, wobei sich ein Kondensator jeweils auf den Spitzenwert des positiven bzw. negativen Eingangssignals auflädt und entlädt, wenn ein kleineres oder kein Signal erscheint. Der auf diese Weise gespeicherte Ist-Wert wird mit einem Soll-Wert verglichen und in Abhängigkeit von der Regelabweichung ein reziprokes Stellglied durch Auf- bzw. Abwärtszählen eines durch einen separaten Oszillator getakteten Zählers nachgeführt.

Aufgrund der Stabilitätsbedingungen sind bei einer derartigen Regeleinrichtung Integrationszeitkonstanten in der Größenordnung von mehr als 30 Sekunden erforderlich. Solche Integrationskonstanten bedingen entweder die Verwendung sehr größer Kapazitäten oder sehr kleiner Ströme, woraus sich bei vielen Anwendungen, bei denen es beispielsweise auf geringen Platzbedarf oder große Langzeitstabilität—wie bei der Anwendung innerhalb von implantierbaren Herzschrittmachern—ankommt, Schwierigkeiten ergeben. Dazu kommt, daß bei einer derartigen Einrichtung die genannten Störsignale großer Amlitude, die sie beispielsweise durch kurzfristige Änderungen des Übergangswiderstandes der Elektroden hervorgerufen werden, eine sehr starke die Empfindlichkeit des Systems herabsetzende Regelwirkung erzeugen und—wegen der großen Regelzeitkonstante—die Ausgangsamplitude der QRS-Komplexe erst nach einem längeren Zeitraum wieder die (beispielsweise zu Synchronisationszwecken erforderliche) Amplitude erreicht.

Nun sind digital arbeitende Amplitudenregelschaltungen für allgemeine oder spezielle andere Anwendungen zwar aus einer Vielzahl von Veröffentlichungen bekannt. Eine derartige schaltung wird aber nur dann optimal arbeiten, wenn sie an die Art der zu verarbeitenden Signale besonders angepaßt ist.

So ist beispielsweise aus der DE—OS 21 58 985 eine Schaltung zur Regelung des Verstärkungsgrades eines Wechselspannungsverstärkers bekannt, bei der—für Anwendungen in der Empfängertecknik-Schwellwertschalter mit monostabilem retriggerbarem Schaltzustand vorgesehen sind, die bei Überschreiten der Schwelle in den instabilen Zustand gesetzt werden und diesen Zustand für einen Zeitraum beibehalten der geringfügig größer ist als die Periodendauer der zugeführten Eingangsspannung, wobei durch den gesetzten Schwellwertschalter eine Torschaltung geöffnet wird, die einen Zähler aktiviert, der ein Verkleinern des Übertragungsfaktors in mehreren Schritten bewirkt.

Bei dieser Anordnung wird zwar auf integrierende Glieder für das Eingangssignal verzichtet. Ein Kapazitäten enthaltendes Zeitglied ist aber mindestens in dem monostabilen Schwellwertschalter enthalten. Außerdem funktioniert die Regelung nur in Richtung auf eine Verkleinerung der Amplituden des Ausgangssignals. Auch wegen der größeren zeitlichen Abstände zwischen aufeinanderfolgenden QRS-Komplexen ist es zur Verarbeitung elektrokardiographischer Signale nicht geeignet.

Der im Anspruch 1 angegebenen Erfindung liegt die Aufgabe zugrunde, eine Amplitudenregelschaltung für elektrokardiographische Signale anzugeben, die ohne größere—entsprechende Kapazitäten—bedingende Zeitkonstanten auskommt, ein Auf- und Abwärtsregeln, ermöglicht und bei einem Optimum an Regelgeschwindigkeit auch bei auftretenden Störsignalen weitgehend unbeeinträchtigt bleibt. Weiterhin soll die Regelschaltung in miniaturisierter Form in Herzschrittmachern einsetzbar und in dessen Signalfluß einbeziehbar sein.

Der Erfindung liegt die Erkenntnis zugrunde, daß bei der angegebenen, jeweils nur auf das Auftreten eines QRS-Komplexes wirksamen Regelung (wenn die erscheinenden Signale nicht die erste, untere Schwelle überschreiten, wird kein Beitrag zu einer Änderung des Übertragungsfaktors geleistet) sich die erforderlichen Schaltungszustände mit optimaler Geschwindigkeit einstellen. Eine integrierende Mittelwertbildung des Eingangssignals ist nicht erforderlich. Da beim Auftreten jedes QRS-Komplexes, dessen Maximalamplitude jeweils innerhalb der durch die betreffenden Schwellen definierten Grenzen liegt, eine Verstärkungsänderung jeweils genau um eine Stufe erfolgt, sind Glieder, welche Signalzustände für einen Zeitraum speichern, um einem Zähler Gelegenheit zu geben durch genügend Takte den Übertragungsfaktor in vielen kleinen Schritten zu verändern nicht erforderlich.

Bei einer vorteilhaften Weiterbildung der Erfindung wird vielmehr der Zeitpunkt der Veränderung des Übertragungsfaktors in einen Zeitraum verlegt, der außerhalb der Zeiten des Erscheinens der für die weitere Signalverarbeitung bedeutsamen Signalanteile liegt, so daß eine Veränderung des Übertragungsfaktors auch in größeren Schritten für das Nutzsignal nicht störend in Erscheinung tritt.

Bei einer anderen vorteilhaften Weiterbildung der Erfindung wird die Gefahr des Auftretens von Instabilitäten durch die besondere Bemessung der Regelschritte im Hinblick auf die daraus resultierende Veränderung des Ausgangssignal wirksam vermieden.

Die Stabilität der Anordnung wird weiter vergrößert, indem dafür gesorgt wird, daß die Amplitudenregelung nicht für den jeweils erfaßten Impuls selbst, sondern erst für nachfolgende Impulse wirksam wird. Damit wird insbesondere eine Rückkopplung verhindert.

Im Zusammenhang mit Anwendungen bei Herzschrittmachern erweist es sich als vorteilhaft, wenn die Änderung des Übertragungsfaktors mit dem Ende der sogenannten Refraktärzeit, die auf einen R-Impuls folgt, vorgenommen wird, so daß die Rückflanke des entsprechenden von dem Zeitgeber erzeugten Impulses als Taktsignal verwendet werden kann.

Dadurch, daß bei einer anderen bevorzugten Ausführung für die Anwendung im Zusammenhang mit künstlichen Herzschrittmachern der Übertragungsfaktor, d.h. die Verstärkung, auf einen Höchstwert heraufgesetzt wird, wenn eine bestimmte Anzahl von Impulsen bzw. die Impulse während einer bestimmten Zeitdauer eine vorgegebene Amplitudenschwelle unterschreiten, ist dafür gesorgt, daß sich eine Synchronisation stets wieder relativ schnell einstellt. Die nach dem Heraufsetzen des Übertragungsfaktors am Ausgang erscheinenden Impulse großer Amplitude erreichen dann nämlich mit Sicherheit die zum Verändern der Verstärkung zu überschreitenden Schwellen. Dazu kommt, daß die beim Anpassen der Regelungseinrichtung in Richtung sich verkleinernder Verstärkung zunächst noch zu großen Amplituden—im Gegensatz zu zu kleinen Amplituden—bei entgegengesetzter Annäherung in angeschlossenen nachfolgenden Anordnungen bereits eine eventuell gewünschte Synchronisation herstellen. Unerwünschte Störsignale beeinträchtigen auch bei zeitweise relativ großer Verstärkung den Betrieb künstlicher Herzschrittmacher nicht, da innerhalb derselben stets Schutzschaltungen gegen Betriebsbeeinflussungen aufgrund derartiger Signale vorgesehen sind. Es wird daher erreicht daß die QRS-Komplexe im Eingangssignal schneller wieder mit ausreichender Amplitude zur Verfügung stehen als es mit der üblichen Änderungsgeschwindigkeit des Übertragungsfaktors einer derartigen Anordnung erreicht werden könnte.

Weiterhin ist es günstig, wenn bei der Anwendung im Zusammenhang mit künstlichen Herzschrittmachern die Stimulationsimpulse selbst bei der Messung ausgeblendet werden. Dadurch können auch die auf Stimulationsimpulse hin erscheinenden Herzsignale zur Regelung ausgenutzt werden.

Andere vorteilhafte Weiterbildungen der Erfindung sind in den Unteransprüchen angegeben und werden anhand einer vorteilhaften Ausführungsform der Erfindung, die in den Zeichnungen dargestellt ist, nachfolgend näher beschrieben. Es zeigen:

Fig. 1 eine Blockschaltung eines Ausführungsbeispiels der erfindungsgemäßen Amplitudenregelungseinrichtung,

Fig. 2 eine detailliertes Schaltbild desselben Ausführungsbeispiels, das zusätzlich einige Signalverbindungen entsprechend einer Anwendung mit einem künstlichen Herzschrittmacher aufweist und

Fig. 3 eine Einzelheit des Ausführungsbeispiels gemäß Fig. 2.

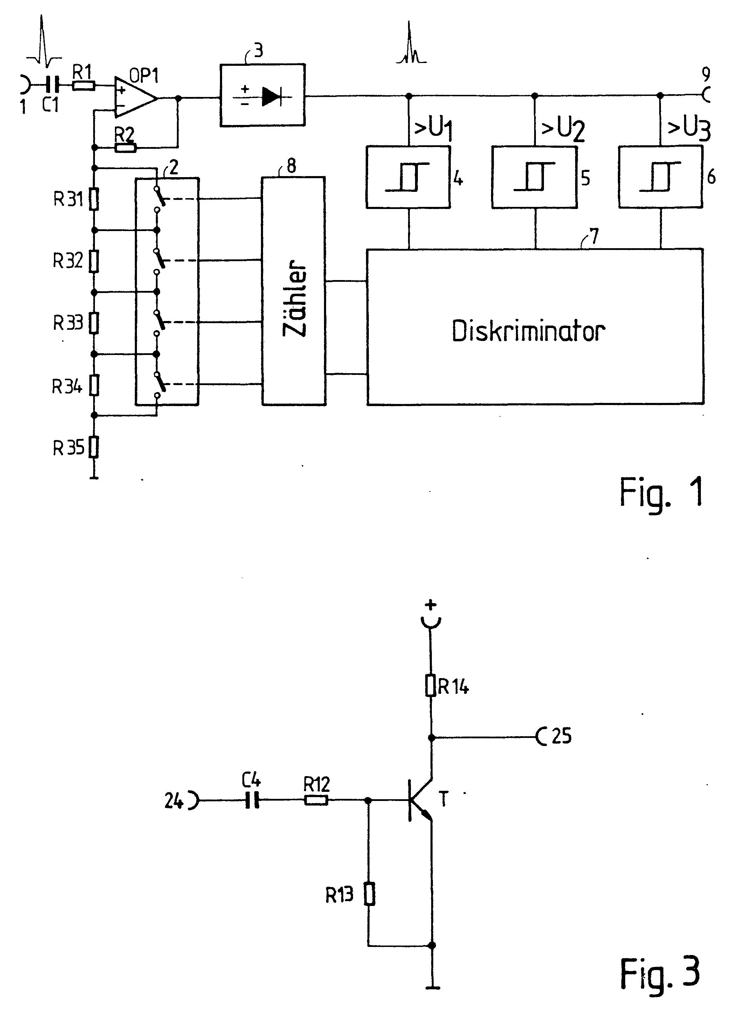

Bei der in Fig. 1 dargestellten Blockschaltung gelangt ein Eingangssignal an den Eingang 1. Dieses Signal bildet die Meßgröße und kann entweder aus dem über Hautelektroden abgeleiteten EKG-Signal oder aus dem über dem im oder am Herzen angebrachte Elektroden abgeleiteten, durch Stimulationsimpulse hervorgerufenen Antwortsignal bestehen.

Das Eingangssignal gelangt über einen Kondensator C1 und einen Widerstand R1 an einen Operationsverstäker OP1, dessen Verstärkungsfaktor mit den erfindungsgemäßen Mitteln geregelt wird. Der Verstärkungsfaktor wird bestimmt durch einen zwischen dessen Ausgang und invertierendem Eingang angeordneten Widerstand R2 und dem zwischen dem invertierenden Eingang und Masse angeordneten Widerstand, der sich aus einer durch eine Widerstandskette R31 bis R35 gebildeten Reihenschaltung von Widerständen zusammensetzt. Die Widerstände R31 bis R34 der Teilerkette sind jeweils mit einem parallelen Schalter versehen. Die Schalter sind dabei in einem separaten Schalterbaustein 2 zusammengefaßt. Der Verstärkungsfaktor des Operationsverstärkers bestimmt sich nach der Formel $v = R2 + R31 + \ldots + R35/R31 + \ldots + R35$. Das Ausgangssignal des Operationsverstärkers OP1 wird einem Zweiweg-Gleichrichter 3 zugeführt, an dessen Ausgang die Impulse des QRS-Signals mit einer einzigen Polarität erscheinen.

Das gleichgerichtete Signal gelangt an drei Schwellendetektoren 4, 5 und 6, die jeweils beim Überschreiten eines vorgegebenen Spannungswertes ein Signal abgeben, wobei die Schematisch angedeutet Hysteresewirkung gering ist und zur Vermeidung von Instabilitäten dient. Die bei den Detektoren 6, 5 und 4 zu überschreitenden Schwellenspannungen verhalten sich wie 40:70:90 und sind auf einen von nachfolgenden Schaltungen optimal zu verarbeitenden Pegel von 100 bezogen.

In einem weiter unten noch näher darzustellenden Diskriminator 7 wird in Abhängigkeit von den Ausgangssignalen der Schwellendetektoren 4 bis 6 der Zählerstand eines Zählers 8 verändert, der seinerseits den Zustand der Schalter im Schalterbaustein 2 beeinflußt.

Bei dem Zähler 8 handelt es sich vorzugsweise um einen Binärzähler, so daß bei entsprechender Stufung der Widerstände R31 bis R34 sich über die vier Schalter des Bausteins 2 insgesamt 16 verschiedene Verstärkungsstufen einstellen lassen.

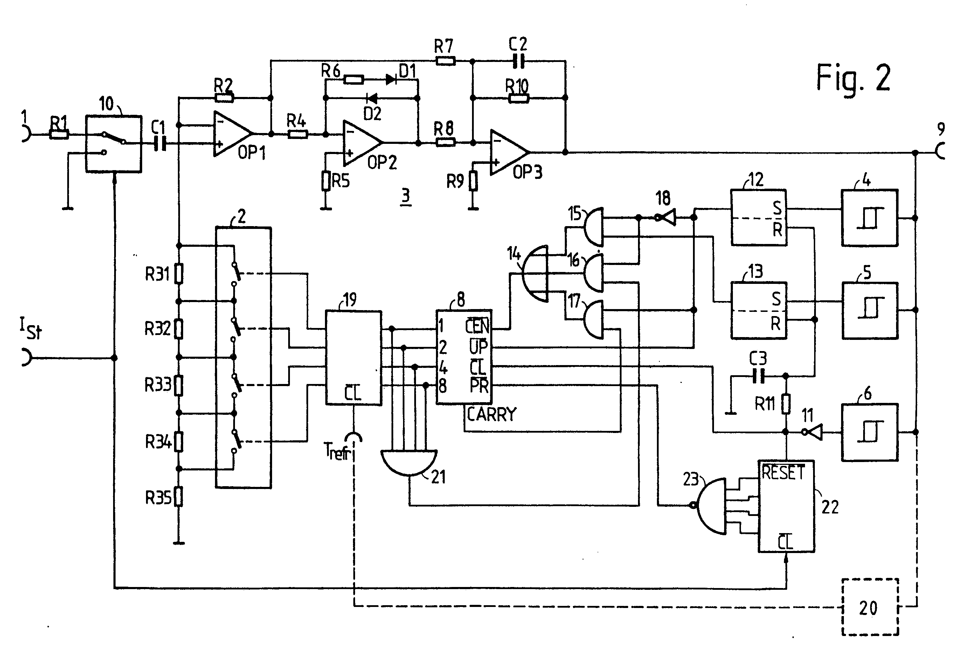

Das gleichgerichtete QRS-Signal steht an einem Ausgang 9 der Schaltung für die Synchronisation eines Herzschrittmachers zur Verfügung. Die Wirkungsweise des Diskriminators 7 soll im einzelnen anhand des nachfolgend beschriebenen in Fig. 2 wiedergegebenen detaillierten Schaltbilds der erfindungsgemäßen Amplitudenregeleinrichtung erfolgen.

Das über am Körper des Patienten angebrachte Elektroden abgeleitet Signal gelangt über den Eingang 1 zu einem Widerstand R1, der mit einem Clamp-Schalter 10 verbunden ist, mittels dessen—gesteuert über einen Eingang $I_{st}$—der mit dem nicht invertierenden Eingang des Operationsverstärkers OP1 verbundene Kondensator C1 zeitweise an ein Bezugspotential gelegt werden kann. Hierdurch werden bei Herzschrittmacheranwendungen, wenn die vom Herzen abgegebenen Signale als Regelgröße dienen, die Schrittmacher-Stimulationsimpulse selbst im Sinne einer Austastung von der Regeleinrichtung ferngehalten, da diese durch ihre große Amplitude die Einstellung eines falschen Verstärkungswertes bewirken würden. Der Operationsverstärker OP1 ist mit den Widerständen R2 und R31 bis R35 in bekannter Weise als nicht invertierender Verstärker geschaltet. Der die Verstärkung des Operationsverstärkers OP1 beeinflussende Schalterbaustein 2 wird weiter unten näher beschrieben.

Das Ausgangssignal des Operationsverstärkers OP1 gelangt an den aus weiteren Operationsverstärkern OP2 und OP3 bestehenden Zweiweg-Gleichrichter 3. Die Beschaltung der letztgenannten Operationsverstärker mit Widerständen R4 bis R10, Dioden D1 und D2, sowie einem Kondensator C2 ist (in bekannter Weise) derart; daß dem Ausgangssignal des Operationsverstärkers OP1 mittels dem als linearer Addierer geschalteten Operationsverstärkers OP3 das Ausgangssignal des als Einweg-Gleichrichter geschalteten Operationsverstärkers OP2 mit doppelter Amplitude und negativem Betrag überlagert wird, so daß sich am Ausgang des Operationsverstärkers OP3 und damit am Ausgang 9 insgesamt ein Ausgangssignal ergibt, das einer Zweiweg-Gleichrichtung der QRS-Signale entspricht. Dieses gleichgerichtete signal wird den Schwellendetektoren 4, 5 und 6 zugeführt, welche, wenn das gleichgerichtete Signal die genannten Schwellwerte überschreitet, jeweils eine abfallende Signalflanke an ihrem Ausgang erzeugen. Die Zuordnung ist dabei so gewählt, daß dem Schwellendetektor 4 die höchste (90%) Schwelle entspricht, während der Schwellendetektor 6 beim Passieren der niedrigsten Schwelle ein Signal abgibt.

Erscheint am Ausgang des Operationsverstärkers OP3 die ansteigende Flanke eines Impulses, so wird zunächst die dem Detektor 6 zugeordnete Schwelle überschritten. Durch das Überschreiten der ersten Schwelle, die sich vorteilhafterweise bei 40% der Impulsamplitude befindet, für die die am Ausgang 9 angeschlossene nachfolgende Schaltung ausgelegt ist, wird noch kein Schaltvorgang ausgelöst. Das ist erst dann der Fall, wenn die Ausgangsspannung des Operationsverstärkers OP3 die genannte Schwelle wieder unterschreitet. Durch die weiter ansteigende Flanke des Impulses wird über Ausgangssignale der Schwellendetektoren 4 (90%) und 5 (70%) durch eine abfallende Flanke am jeweiligen Setz-Eingang S ein zugeordnetes RS-Flip-flop 12 bzw. 13 gesetzt, so daß nach Erreichen des Maximums festgehalten ist, welche Schwellenwerte vom Impuls überschritten wurden. Erreicht die Rückflanke des Impulses wieder die dem Schwellendetektor 6 zugeordnete niedrigste Amplitudenschwelle, wird der Zähler 8, der aufwärts und abwärts zählen kann, je nach der erreichten Schwelle, um einen Werte aufwärts oder abwärts gesetzt bzw. er behält seinen Wert bei. Die Rückflanke des Impulses vom ·Inverter 11 gelangt dazu an einen CLOCK-Eingang $\overline{CL}$ des Zählers 8, wobei über zusätzliche Eingänge (Zählrichtung $\overline{UP}$ und Toreingang $\overline{CEN}$) darüber entschieden wird, in welcher Richtung bzw. ob überhaupt gezählt wird. Ein Signal am Eingang UP schaltet den Zähler auf Abwärtsrichtung, während ein Signal am Eingang $\overline{CEN}$ den Eingang $\overline{CL}$ einschaltet und somit eine Veränderung des Zählerstandes zuläßt. (Die Kennzeichnung der Bezeichnung der Eingänge mit einem Querstrich bedeutet, daß diese sich auf negative Logiksignal beziehen bzw. im Falle der Clock-Eingänge $\overline{CL}$ auf Impulsrückflanken ansprechen).

Die aus einem ODER-Gatter 14, drei UND-Gattern 15, 16 und 17, sowie einem Inverter 18 bestehende Logikschaltung erzeugt die im folgenden beschriebenen Zuordnungen:

Überschreitet ein Impulsmaximum lediglich die niedrigste dem Schwellendetektor 6 zugeordnete Schwelle, so wird der Zählerstand durch die Rückflanke des an den $\overline{\text{CL}}$-Eingang gelangten Impulses um eine Stufe heraufgesetzt, da der Zähler weder über seinen Eingang $\overline{\text{UP}}$ auf Abwärtsrichtung geschaltet ist noch sein $\overline{\text{CL}}$-Eingang über den Eingang $\overline{\text{CEN}}$ gesperrt wurde.

Erreicht und überschreitet das Amplitudenmaximum auch die dem Schwellendetektor 5 zugeordnete Amplitudenschwelle, so wird das RS-Flip-Flop 13 gesetzt, d.h. sein Ausgang geht in den H-Zustand über. Dieses Signal gelangt über das UND-Gatter 15, dessen anderer Eingang ebenfalls auf H-Potential liegt und das ODER-Gatter 14 zum $\overline{\text{CEN}}$-Eingang des Zählers 8, so daß der $\overline{\text{CL}}$-Eingang des Zählers 8 gesperrt ist und damit dieser nicht über die Rückflanke des vom Schwellwertdetektor 6 stammenden Impulses getaktet werden kann.

Wurde auch die dem Schwellendetektor 4 zugeordnete Amplitudenschwelle überschritten, wird das Flip-Flop 12 ebenfalls gesetzt, so daß dessen in den H-Zustand gelangender Ausgang über den Inverter 18 das UND-Gatter 15 sperrt und damit das Flip-Flop 13 wirkungslos wird. Der Zähler 8 ist daraufhin nicht mehr für die vom Schwellendetektor 6 an seinen $\overline{\text{CL}}$-Eingang gelangenden Taktimpulse gesperrt. Da zusätzlich vom Ausgang des Flip-Flops 12 der H-Zustand an den Eingang UP des Zählers 8 gelangt, wird dessen Zählrichtung auf Abwärtszählen umgeschaltet.

Die Ausgänge des Zählers 8 sind mit einer Latch-Stufe 19 verbunden welche, durch einen an ihren $\overline{\text{CL}}$-Eingang gelangenden Impuls getaktet, die Zustände an den mit 1 bis 8 bezeichneten Ausgängen des Zählers 8 zu dem Schalterbaustein 2 gelangen läßt. Je nach augenblicklichem Stand des Zählers 8 werden die den binären Ziffern mit den Werten $2^0$ bis $2^3$ (entsprechend 1 bis 8) zugeordneten Widerstände 31 bis 34, die gemäß ihrer binären Wertigkeit gestuft sind durch Schließen des jeweils parallel dazu angeordneten Schalters überbruckt. Die Verstärkung des Operationsverstärkers OP1 ist nach der oben angegebenen Beziehung um so größer, je kleiner der Gesamtwiderstand der Teilerkette zwischen seinem invertierenden Eingang und dem Bezugspotential ist. Die Vergrößerung der Verstärkung durch Kurzschließen des zugeordneten Widerstandes entspricht dabei dessen Wert.

Ein Aufwärtszählen entspricht damit einer Heraufsetzung der Verstärkung, während ein Abwärtszählen zu deren Herabsetzung führt. Nachdem jeweils durch die Rückflanke des Schwellendetektors 6 in Abhängigkeit vom Zustand der Flip-Flops 12 und 13 ein Zählvorgang ausgeführt wurde, erfolgt unmittelbar anschließend—verzögert durch eine aus einem Widerstand R11 und einem Kondensator C3 bestehenden RC-Kombination—ein Rücksetzen der Flip-Flops 12 und 13 über deren R-Eingänge in ihren Ausgangszustand.

Das Übertragen des Zählerstandes zum Schalterbaustein 2 erfolgt—wie erwähnt—über die Latch-Stufe 19, der über ihren $\overline{\text{CL}}$-Eingang ein mittels eines Monoflops 20 (gestrichelt dargestellt) verzögerter Ausgangsimpuls zugeführt wird. Auf diese Weise wird erreicht, daß die Übertragung der im QRS-Komplex enthaltenen Impulse nicht von deren eigener Amplitude abhängig ist, sondern stets nur von derjenigen vorangehender Komplexe beeinlußt wird.

Bei Anwendungen für Herzschrittmacher ist das notwendige Monoflop zur Unterdrückung von Impulsen innerhalb der auf einen R-Zacken folgenden Refraktärzeit $T_{refr}$ ohnehin vorhanden. Die Rückflanke eines zu einer derartigen Signalunterdrückung im Schrittmacher herangezogenen Impulses kann deshalb in vorteilhafter Weise zur Auslösung der geschilderten Signalvorgänge dienen.

Die vorgesehen Logikelemente sorgen außerdem dafür, daß der Zähler 8 nicht über seinen höchsten Stand hinaus aufwärts bzw. über seinen niedrigsten Stand hinaus abwärts zählen kann, da damit die Regelung beeinträchtigende Sprünge des Verstärkungswertes verbunden wären. Erreicht der Zähler seinen höchsten Stand—d.h. alle Ausgänge sind auf H-Potential—so nimmt der Ausgang eines UND-Gatters 21 ebenfalls H-Potential an. Damit geht der Ausgang des UND-Gatters 16 ebenfalls in den H-Zustand über, wenn sein anderer Eingang ein entsprechendes Potential aufweist. Das ist genau dann der Fall, wenn das Flip-Flop 12 nicht gesetzt ist, der Zähler also nicht auf Abwärtszählen geschaltet ist. Über das ODER-Gatter 14 und den Eingang $\overline{\text{CEN}}$ wird infolgedessen der Takteingang des Zählers gesperrt. Entsprechend wird ein weiteres Abwärtszählen verhindert, wenn alle Ausgänge des Zählers L-Potential aufweisen. In diesem Fall gibt der Übertragungsausgang CARRY des Zählers 8 ein Signal ab, das über das UND-Gatter 17 und das ODER-Gatter 14 den Zähler in entsprechender Weise sperrt, wenn das Flip-Flop 12 gesetzt ist, d.h. der nächste Taktimpuls einen weiteren Zählvorgang auslösen würde. Es ist damit sichergestellt, daß die Verstärkung des Operationsverstärkers OP1 nur innerhalb der vorgesehen Grenzen mit kleinen Sprungweiten veränderlich ist.

Eine bei linear arbeitenden Reglern nicht auszuschließende Instabilität läßt sich bei der erfindungsgemäßen Regelvorrichtung in einfacher Weise verhindern: die Differenzen der den Schwellendetektoren zugeordneten Amplitudenschwellen werden so gewählt, daß sie kleiner sind als die von aus der Verstärkungsänderung um eine Stufe resultierenden Veränderung der Amplitude der Impulse am Ausgang des Operationsverstärkers OP3. Da die absoluten Amplituden der Ausgangsimpulse sich durch die erfindungsgemäße Regelung innerhalb relativ enger Grenzen halten, liegen auch die aus der Änderung der Verstärkung resultierenden Amplitudenänderungen in einem relativ engen Bereich, so daß auf diese Weise der Fall, daß eine durch eine Verstärkungsänderung hervorgerufene Änderung der Amplitude der Ausgangimpulse sofort wieder eine erneute Verstärkungsänderung hervorruft und dadurch eine Instabilität eintritt, vermieden ist.

Da beim Auftreten von die höchste Schwelle überschreitenden (Stör-)impulsen stets pro Impuls—unabhängig von dessen Amplitudenur eine Verstärkungsänderung um eine Stufe erfolgen kann, bleiben Störspitzen auch großer Amplitude weitgehend ohne Einfluß auf die Regelung.

Von besonderer Bedeutung ist der Fall, daß für einen Zeitraum die Amplituden der QRS-Impulse nicht die unterste Schwell überschreiten. Das kann bei einen starken Absinken der zur Verfügung stehenden Signalamplitude oder im Leerlauf der Fall sein. Hier geht es darum, recht bald wieder ein für die nachfolgende Auswertung geeignetes EKG-Signal zur Verfügung zu stellen. Für Synchronisationszwecke müssen die QRS-Komplexe dabei eine ausreichende Amplitude aufweisen. Um bei einem derartigen Signalausfall unter Umgehung der schrittweisen Vergrößerung der Verstärkung sofort nach dem Erscheinen von QRS-Komplexen mit verwertbarer Amplitude ein entsprechendes Ausgangssignal zu gewährleisten, wird einige Zeit nachdem kein die untere Schwelle überschreitendes Signal mehr erschien, der Übertragungsfaktor auf einen Maximalwert heraufgesetzt.

Zur Ermittlung dieses Zeitraums des "Signalausfalls" dient ein weiterer Zähler 22, der bei Anwendung mit einem künstlichen Demand-Schrittmacher durch die künstlichen Stimulationsimpulse in dessen asynchroner Grundfrequenz getaktet wird, die am Eingang $I_{st}$ erscheint, wenn keine herzeigenen QRS-Komplexe festgestellt werden. (In anderen Anwendungen kann auch jedes geeignete Taktsignal verwendet werden, welches eine Zeitbasis darstellen kann oder der Zähler 22 durch ein Monoflop als Zeitgeber ersetzt werden). Da der Rucksetz-Eingang $\overline{RESET}$ über den Inverter 11 mit dem Ausgang des Schwellendetektors 6 verbunden ist, wird jedesmal zurückgesetzt, wenn dieser Schwellendetektor anspricht, d.h. ein Impuls die zugeordnete Schwellenamplitude überschreitet.

Erreicht der Zähler 22 nach einiger Zeit, die durch die Vorgesehene Anwendung bestimmt wird und in der Regel einige Herzschläge umfaßt, einen vorgegebenen Stand (im dargestellten Beispiel binär 1111), so setzt er den Zähler 8 mittels eines NAND-Gatters 23 über seinen Eingang $\overline{PR}$ auf die binäre Zahl 1111, so daß die Verstärkung des Operationsverstärkers OP1 ihren höchsten Wert erreicht. Der Zähler 8 wird jeweils auf seinen der höchsten Verstärkung des Operationsverstärkers OP1 entsprechenden Zählerstand gesetzt, wenn innerhalb eines vorgegebenen Zeitraums am Ausgang des Schwellendetektors 6 kein Impuls auftritt.

Nachdem die Schaltung beim Ausfall von Impulsen ausreichenden Amplitude sich auf höchste Verstärkung einstellt, ist gewährleistet, daß bei typischen Fehlern bei der Ableitung von QRS-Impulsen, beispielsweise der instabilen Befestigung der zur Signalaufnahme verwendeten Elektrode, nach kürzester Zeit die Synchronisation wiederhergestellt wird, da nach einem derartigen Signalausfall umgehend—von den Werten höchster Verstärkung her—eine Anpassung an die neuen Übertragungsverhältnissen erfolgt. Wegen der Eigenschaft von Schrittmacherschaltungen nur auf Signalimpulse zu reagieren, die—entsprechen dem Auftreten von QRS-Impulsen im natürlichen Herzsignal—in vorgegebenen Mindestzeitasbständen erscheinen, werden diese von eventuell durch die vergrößerte Verstärkung in ihrer Amplitude heraufgesetzten Störimpulsen nicht beeinträchtigt, da diese gewöhnlich in einer anderen zeitlichen Folge erscheinen. Stellt sich anschließen wieder ein normaler Betriebszustand ein, werden auftretende Herzsignale mit Sicherheit erfaßt und es stellt sich schnell wieder der zugehörige Übertragungsfaktor ein, wobei die nachfolgenden Stufen in der Regel besser in der Lage sind, amplitudenmäßig zu große Signale zu verarbeiten als es bei zu kleinen Signalen der Fall wäre.

In Fig. 3 ist ein besonders einfaches Ausführungsbeispiel eines gemäß der Erfindung verwendbaren Schwellwertdetektors dargestellt, wie er in der Schaltung gemäß Fig. 2 vorgesehen sein kann.

Von einem Eingang 24 gelangt das gleichgerichtete Eingangssignal über einen eine bleibende Gleichspannungsabweichung kompensierenden Kondensator C4 mit einer zur impulsgetreuen Übertragung der Signale ausreichend großen Kapazität an einen aus Widerständen R12 und R13 gebildeten Spannungsteiler. Parallel zu dem Widerstand R13 ist die Basis-Emitter-Strecke eines Transistors T geschaltet, der leitend wird, wenn der Spannungsabfall am Widerstand R13 die Schwellspannung des Transistors überschreitet. Der dabei durch den Kollektorwiderstand R14 fließende Strom erzeugt einen Spannungssprung am Ausgangsanschluß 25, wobei sichentsprechend der Darstellung gemäß Fig. 2— beim Überschreiten der vorgegebenen Amplitudenschwelle eine abfallende Impulsflanke ergibt. Die jeweilige Schwellspannung läßt sich unter Berücksichtigung der Basis-Emitter-Spannung des Transistors durch das Verhältnis der Widerstände R12/R13 festlegen. Diese einfache Ausbildung eines Schwellwertdetektors ist insbesondere Bei Anwendungen in implantierbaren Herzschrittmachern vorteilhaft, da sich hier die Temperaturabhängigkeit der Schwellspannung der Basis-Emitter-Strecke des verwendeten Transistors wegen der ohnehin nahezu konstanten Körpertemperatur nicht auswirken kann.

Die dargestellte Ausführungsform stellt nur eine Möglichkeit der Realisierung der Erfindung dar, wobei die praktische Ausführung wesentlich von der Art der zur Verfügung stehenden Bauelemente abhängig ist. So ergeben sich beispielsweise bei der Verwendung von integrierten Schaltungselementen in CMOS-Technik Änderungen bezüglich der Logik-Gatter, wenn eine Realisierung mit möglichst wenigen, allgemein erhältlichen Bauelemente angestrebt wird. Dabei sind auch die Eigenschaften der Zählerbauelemente (bezüglich ihrer Fähigkeiten auf- und abwärts zu zählen sowie über separate Eingänge gesteuert vorbestimmte Zählerzustände anzunehmen) zu berücksichtigen. Es besteht dabei ebenefalls die Möglichkeit, die erfindungsgemäße Schaltungsanordnung im Rahmen einer von einem Mikroprozessor gesteuerten Schaltungsanordnung zu realisieren.

**Patentansprüche**

1. Schaltungsanordnung für ein elektrokardiographisches, QRS-Signale enthaltendes Signal mit digital arbeitender Amplitudenregelung, bei der ein Verstärker (OP1) vorgesehen ist, dessen Übertragungsfaktor schwellwertabhängig stufenweise regelbar ist, dadurch gekennzeichnet, daß dem Verstärker (OP1) eine Gleichrichterschaltung (3) in der Weise nachgeschaltet ist, daß sich eine Zweiweg-Gleichrichtung der QRS-Signale ergibt, und daß die gleichgerichteten QRS-Signale drei Schwellwertdetektoren (4 bis 6) zugeführt werden, die jeweils auf verschiedene Schwellwerte ($U_1$, $U_2$, $U_3$) ansprechen, wobei jedes Mal, wenn die erste Schwell ($U_3$) überschritten wird, der Übertragungsfaktor um eine Verstärkungsstufe (R31 bis R35) heraufgesetzt wird, sofern die zweite Schwelle ($U_2$) nicht überschritten ist, bzw. der Übertragungsfaktor um eine Verstärkungsstufe herabgesetzt wird, sofern auch die dritte Schwelle ($U_1$) überschritten ist.

2. Schaltungsanodnung nach Anspruch 1, dadurch gekennzeichnet, daß die Ausgänge der Schwellwertdetektoren (4 bis 6) mit bistabilen Schaltelementen (12, 13) verbunden sind, welche das Überschreiten der betreffenden Schwellen durch das Signal festhalten und über logische Verknüpfungsglieder (14 bis 18) eine Änderung des Übertragungsfaktors, entsprechend der höchsten überschrittenen Schwelle, erste dann veranlassen, wenn die niedrigste Schwelle durch die gleichgerichteten QRS-Signale wieder unterschritten worden ist.

3. Schaltungsanordnung nach einem der vorangehenden Ansprüche, dadurch gekennzeichnet, daß die Differenz zwischen der zweiten und der dritten Schwelle größer ist als der Betrag der Vergrößerung bzw. Verkleinerung der Amplitude des Spitzenwertes des QRS-Signals am Schaltungsausgang (9) der durch die Änderung des Übertragungsfaktors bei konstantem Eingangssignal um eine Stufe bewirkt wird.

4. Schaltungsanordnung nach einem der vorangehenden Ansprüche, dadurch gekennzeichnet, daß die den drei Schwellen zugeordneten Amplitudenwerte sich im wesentlichen wie 40 zu 70 zu 90 verhalten, wobei 100 der Sollamplitude der Spitzenwerte des QRS-Signals entspricht.

5. Schaltungsanordnung nach einem der vorangehenden Ansprüche, dadurch gekennzeichnet, daß ein zusätzlicher Schaltkreis (22, 23) vorgesehen ist, welcher, wenn die Spitzenwerte der Signale für eine Zeitdauer, die einem Zeitraum entspricht, innerhalb dessen mehrere natürliche QRS-Signale zu erwarten wären, die erste Schwelle nicht überschreiten den Übertragungsfaktor auf einen Maximalwert heraufsetzt.

6. Schaltungsanordnung nach Anspruch 5 für einen künstlichen Herzschrittmacher, dadurch gekennzeichnet, daß der zusätzliche Schaltkreis einen Zähler (22) für die vom Herzschrittmacher abgegebenen Stimulationsimpulse enthält, der jeweils durch ein die erste Schwelle überschreitendes Signal zurückgesetzt wird und beim Erreichen eines vorgegebenen Zählerstands den Übertraguungsfaktor auf den Maximalwert heraufsetzt.

7. Schaltungsanordnung nach einem der vorangehenden Ansprüche, dadurch gekennzeichnet, daß für eine verzögerte Änderung des Übertragungsfaktors ein Verzögerungsglied (20) vorgesehen ist, das die Änderung des Übertragungsfaktors erst nach einem Zeitraum auslöst, der dem im QRS-Signal enthaltenen T-Impuls entspricht.

8. Schaltungsanordnung nach Anspruch 7 zur Anwendung in künstlichen Herzschrittmachern, dadurch gekennzeichnet, daß das Verzögerungsglied (20) so ausgebildet ist, daß es für die Dauer der Refraktärzeit in der Schaltung des Herzschrittmachers die Abgabe von Stimulationsimpulsen unterdrückt.

9. Schaltungsanordnung nach einem der vorangehenden Ansprüche zur Anwendung in einem künstlichen Herzschrittmacher, dadurch gekennzeichnet, daß eine Austastschaltung (10) vorgesehen ist, welche den Eingang des Verstärkers (OP1) für die Dauer der vom Herzschrittmacher abgebenen Stimulationsimpulse sperrt.

10. Schaltungsanordnung nach einem der vorangehenden Ansprüche zur Anwendung in einem künstlichen Herzschrittmacher, dadurch gekennzeichnet, daß als Schwellwertdetektor die Basis-Emitter-Strecke eines Transistors (T) als Referenzelement für die über einen ohmschen Spannungsteiler herabgesetzte Spannung vorgesehen ist, wobei der Spannungsteiler gleichzeitig als Basisspannungsteiler dient.

## Revendications

1. Montage pour un signal électrocardiographique contenant des signaux QRS, comportant une régulation numérique d'amplitude, pour laquelle est prévu un amplificateur (OP1) dont le facteur de transfert est réglable en continu en fonction d'une valeur de seuil, caractérisé en ce qu'un circuit redresseur (3) est monté de telle manière à la suite de l'amplificateur (OP1) que les signaux QRS sont soumis à un redressement à double voie, et que les signaux QRS redressés sont appliqués à trois détecteurs de seuil (4 à 6) qui réagissent chacun à une valeur de seuil différente ($U_1$, $U_2$, $U_3$), l'agencement étant tel qu'à chaque dépassement du premier seuil ($U_3$), le facteur de transfert est relevé, d'un étage d'amplification (R31 à R35) si le second seuil ($U_2$) n'est pas dépassé, et le facteur de transfert est abaissé d'un étage d'amplification si le troisième seuil ($U_1$) est également dépassé.

2. Montage selon la revendication 1, caractérisé en ce que les sorties des détecteurs (4 à 6) sont reliées à des éléments de commutation bistables (12, 13) qui mémorisent le dépassement des seuils concernés par le signal et qui déclenchent seulement un changement du facteur de transfert, à travers des éléments logiques (14 à 18) et suivant le seuil le plus élevé qui a été dépassé, lorsque les signaux QRS redressés redescendent sous le seuil le plus bas.

3. Montage selon une des revendications précédentes, caractérisé en ce que l'écart entre le second et le troisième seuil est plus grande que l'augmentation ou la diminution de l'amplitude de la valeur de crête du signal QRS, à la sortie (9) due montage, produite par le changement du facteur de transfert d'un étage, à signal d'entrée constant.

4. Montage selon une des revendications précédentes, caractérisé en ce que les valeurs d'amplitude associées aux trois seuils présentent sensiblement les rapports 40:70:90, l'amplitude de consigne de la valeur de crête du signal QRS correspondant à 100.

5. Montage selon une des revendications précédentes, caractérisé en ce qu'il comprend un circuit de commutation supplémentaire (22, 23) qui relève le facteur de transfert à une valeur maximale lorsque les valeurs de crête des signaux ne dépassent pas le premier seuil pendant une durée au cours de laquelle on pourrait s'attendre à plusieurs signaux QRS naturels.

6. Montage selon la revendication 5 pour un stimulateur cardiaque artificiel, caractérisé en ce que le circuit de commutation supplémentaire contient un compteur (22) pour les impulsions de stimulation délivrées par le stimulateur cardiaque, compteur qui est chaque fois remis à zéro par un signal dépassant le premier seuil et qui, lorsqu'il atteint une position de comptage préfixée, relève le facteur de transfert à la valeur maximale.

7. Montage selon une des revendications précédentes, caractérisé en ce que, pour retarder le changement du facteur de transfert, il comporte un élément de retard (20) qui déclenche le changement de facteur seulement après une durée correspondant à l'impulsion T contenue dans le signal QRS.

8. Montage selon la revendication 7 destiné à être appliqué dans des stimulateurs cardiaques artificiels, caractérisé en ce que l'élément de retard (20) est réalisé de manière qu'il supprime la délivrance d'impulsions de stimulation dans le montage du stimulateur cardiaque pendant la durée de la période réfractaire.

9. Montage selon une des revendications précédentes et destiné à être utilisé dans un stimulateur cardiaque artificiel, caractérisé en ce qu'il comporte un circuit de suppression (10) qui bloque l'entrée de l'amplificateur (OP1) pendant la durée des impulsions de stimulation délivrées par le stimulateur cardiaque.

10. Montage selon une des revendications précédentes et destiné à être utilisé dans un stimulateur cardiaque artificiel, caractérisé en ce qu'un détecteur est formé par le circuit base-émetteur d'un transistor (T) comme élément de référence pour la tension réduite par un diviseur de tension ohmique, lequel sert en même temps de diviseur de la tension de base.

## Claims

1. Circuit arrangement for an electrocardiographic signal containing QRS-signals and with digitally operating amplitude control, in which there is provided an amplifier (OP1) of which the transfer factor is adjustable in stages in dependance on a threshold value, characterised in that a rectifier circuit (3) is connected after the amplifier (OP1) such that there results a full-wave rectification of the QRS-signals, and that the rectified QRS-signals are supplied to three threshold value detectors (4 to 6), which respectively respond to different threshold values ($U_1$, $U_2$, $U_3$) so that each time that the first threshold ($U_3$) is exceeded the transfer factor is increased by one amplification step (R31 to R35), provided that the second threshold ($U_2$) is not exceeded, and/or the transfer factor is reduced by one amplification stage, provided also that the third threshold ($U_1$) is exceeded.

2. A circuit arrangement according to claim 1, characterised in that the outputs of the threshold value detectors (4 to 6) are connected to bi-stable circuit elements (12, 13) which hold back the exceeding of the corresponding thresholds by the signal and, by means of logic elements (14 to 18) only cause an alteration of the transfer factor corresponding to the highest exceeded threshold when the rectified QRS-signal have again fallen below the lowest threshold.

3. A circuit arrangement according to any one of the preceding claims, characterised in that the difference between the second and the third thresholds is larger than the amount of the increase or decrease of the amplitude of the peak value of the QRS-signal at the output (9) of the circuit which is caused by the alteration of the transfer factor by one stage with a constant input signal.

4. A circuit arrangement according to any one of the preceding claims, characterised in that the amplitude values associated with the three thresholds are essentially in the relationship 40 to 70 to 90, where 100 corresponds to the desired amplitude of the peak values of the QRS-signal.

5. A circuit arrangement according to any one of the preceding claims, characterised in that there is provided an additional circuit (22, 23), which raises the transfer factor to a maximum value when the peak values of the signals do not exceed the first threshold for a length of time which corresponds to a period of time within which a plurality of natural QRS-signals would be expected.

6. A circuit arrangement according to claim 5 for an artificial heart pacemaker, characterised in that the additional circuit contains a counter (22) for the stimulation pulses emitted by the heart pacemaker, which in each case is reset by a signal exceeding the first threshold and upon reaching a predetermined count raises the transfer factor to the maximum value.

7. A circuit arrangement according to any one of the preceding claims, characterised in that for a delayed adjustment of the transfer factor there is provided a delay element (20) which only triggers the adjustment of the transfer factor after a period of time which corresponds to the T-pulse included in the QRS-signal.

8. A circuit arrangement according to claim 7 for use in artificial heart pacemakers, characterised in that the delay element (20) is so constructed that for the duration of the refractory time it suppresses the emission of stimulation pulses in the circuit of the heart pacemaker.

9. A circuit arrangement according to any one of the preceding claims for use in an artificial heart pacemaker, characterised in that there is provided a blanking circuit (10) which for the duration of the stimulation pulses emitted by the heat pacemaker blocks the input of the amplifier (OP1).

10. A circuit arrangement according to any one of the preceding claims for use in an artificial heart pacemaker, characterised in that as threshold value detector there is provided the base-emitter path of a transistor (T) as reference element for the voltage reduced by means of an ohmic voltage divider, the voltage divider simultaneously serving as base voltage divider.

Fig. 1

Fig. 3

Fig. 2